# EUROPEAN PATENT SPECIFICATION

(11) **EP 4 380 740 B1**
(45) Date of publication and mention of the grant of the patent: **09.12.2026**
(21) Application number: 22856395.3
(22) Date of filing: 14.07.2022
(51) Int. Cl.: B29C 49/48, B29C 49/14, A61F 2/95, B21D 39/04, A61F 2/958, B29C 55/22, A61M 25/10, B29C 49/64, B29C 51/00, B29K 105/00, B29L 31/00

(54) **PARISON FORMER**
VORFORMLINGFORMER
DISPOSITIF DE FORMATION DE PARAISON

(30) Priority: 07.08.2021 US 202163230698 P
(43) Date of publication of application: 12.06.2024
(73) Proprietor: Blockwise Engineering LLC, Tempe, AZ 85281 (US)
(72) Inventor: BOYD, Jordan, Bradley, Phoenix, AZ 85018 (US); GOFF, Ed, Phoenix, AZ 85018 (US); WARRINER, Jeremiah, J., Tempe, AZ 85281 (US)
(74) Representative: Noble, Frederick
(86) International application number: PCT/US2022/037138
(87) International publication number: WO 2023/018509

(56) References cited:
- US-A- 5 057 267
- US-A- 5 351 462
- US-A1- 2006 150 669
- US-A1- 2009 001 633
- US-B1- 8 043 553
- US-B1- 8 043 553
- US-B1- 9 956 604
- US-B2- 7 743 481

## Description

### BACKGROUND OF THE INVENTION

### Field of the Invention

The invention relates to a parison former and method of forming a parison using said parison former.

### Background

Medical balloons are used in a wide variety of medical procedures including, but not limited to angioplasty, stent placement and expansion, stent graft placement and expansion and the like. Parison tubes are hollow plastic tubes used as raw material to form medical balloons. Parison manufacturing machines are slower than desired and often produce balloon portions with tapered ends that are longer than desired. A shorter tapered end may be desired as it provides better accuracy and clarity of an expanded zone when forming the medical balloon.

US 7743481 B2 discloses a method of crimping polymeric stents by applying a radial force to the stent to reduce the diameter of the stent and a longitudinal force to elongate the stent. The stent is heated prior to being inserted into a tube. The tube is then stretched, deforming the malleable tube positioned inside the tube. In particular it discloses:
A
   parison tube former comprising: a) a first tube retainer;
b) a second tube retainer;
c) a radial compression heater comprising:
   i) a compression die having a working surface, the compression dies is arranged radially to form a central cavity defined by said working surfaces, wherein the central cavity has a central cavity diameter;
   ii) a heater configured in the compression die and configured to heat the compression die;
   iii) a die actuator configured to move the compression die inward to reduce the central cavity diameter;
d) a preform tube extending through the central cavity of the radial compression heater and having a length between the first tube retainer and the second tube retainer; wherein the radial compression heater is configured to heat a portion of the length of the preform tube between the first tube retainer and the second tube retainer, wherein the compression die is configured to close down onto the outer surface of the preform tube, wherein the working surface of said compression die contact the preform tube to heat a portion of said length of the preform tube via conduction; and wherein the first tube retainer and second tube retainer are configured to move apart from each other to stretch the preform tube and form a parison tube having a balloon portion configured between a first stretched portion and a second stretched portion.

US8043553 B1 relates to methods of manufacturing a medical device, such as an implantable medical device or an inflatable member what includes radial deformation of a polymer tube.

US9956604 B1 discloses a radial compression apparatus comprising a funnel for radially compressing articles, such as stents. The mechanism comprises a plurality of compression dies that move in unison to cause a cylindrical cavity of the funnel to open or close around the article.

### SUMMARY OF THE INVENTION

The invention is directed to a parison tube former and in accordance wit claim 1. An exemplary parison tube former utilizes a radial compression heater to heat a very specific portion of the length of a preform tube for stretching. A radial compression heater has a plurality of compression dies that form a central cavity for receiving a preform tube. The working surfaces of the compression dies close down onto the outer surface of the preform tube to heat the preform tube via conduction. This heating method more accurately and precisely heats the preform tube such that necking of the preform tube during the stretching operation is very controlled. A first stretched portion of the preform tube is produced by stretching the preform tube after heating with the radial compression heater. A second portion of the preform tube is then located within the central cavity and is also heated by the radial compression heater. After the second portion is heated, the preform tube is stretched to produce a second stretched portion of the preform tube and an unexpanded portion of the preform tube, or balloon portion of the parison tube. The tapered ends of the balloon portion may be much sharper or shorted that those of parison tubes made through hot air heating, as the heated zone is not as well controlled as it is through conduction heating. Furthermore, the compression dies may be configured to remain in contact with the preform tube during the stretching operations, wherein the compression dies are forced inward by the die actuator, and may contact the preform tube as the preform tube necks down. Maintaining conducting heating during this stretching operation enables very controlled stretching with less stress concentration in the polymer as a result. The parison tube has a balloon portion that has a larger diameter than the diameter of the first and second stretched portions such as 50% larger or more, 100% larger or more, 150% larger or more, 200% larger or more and any range between and including the values provided.

An exemplary parison preform tube may be made out a polymeric material, such as a plastic and particularly a thermoplastic that can be melted or softened by heating and stretched including, but not limited to, polyethylene, polyester, polyamide, polyvinyl chloride, urethane and the like. A preform tube for the parison or the balloon diameter may be sized for entry into a conduit within the body, such as a vasculature conduit or digestive system conduit and may have a diameter of about 0.5mm or more, 1mm or more, 2mm or more, 5mm or more, 10mm or more, 20mm or more, 40mm or more, 50mm or more and any range between and including the diameters listed. The length of the balloon portion of the parison tube may be configured to expand a stent or stent graft and/or open a vessel within the body, such an artery or vein, or part of the digestive tract including intestine. The length of the balloon portion may be about 2mm or more 5mm or more, 10mm or more, 20mm or more, 40mm or more, 50mm, 75mm or more, 100mm or more and any range between and including the length values provided.

An exemplary parison former incorporates a first and second tube retainer that are used to hold the ends of the preform tube. One or both of the tube retainers move during the stretching operation to move the two tube retainers apart from each other. The tube retainers may be configured on a rail and may move along the rail by a tube retainer actuator. For example, a motor may move the tube retainer linearly along the rail. A rail may be any suitable guide for controlling the motion of the tube retainer along the length axis of the preform tube. An exemplary motor may be a stepper motor having very precise control of the amount of motion produced and the speed of motion, or speed of stretching produced. Furthermore, the preform may be stretched at a constant or variable speed and a stepper motor may enable a programmable rate of stretch to produce a uniform stretched portion with a desired tapered end on the balloon portion.

### BRIEF DESCRIPTION OF SEVERAL VIEWS OF THE DRAWINGS

The accompanying drawings are included to provide a further understanding of the invention and are incorporated in and constitute a part of this specification, illustrate embodiments of the invention, and together with the description serve to explain the principles of the invention.
Figure 1 shows a perspective view of an exemplary preform tube that is used to form a parison tube having a balloon portion.
Figure 2 shows a side view of an exemplary parison tube having a balloon portion configured between a first stretched portion and a second stretched portion.
Figures 3 to 6 show an exemplary parison former forming a parison tube.

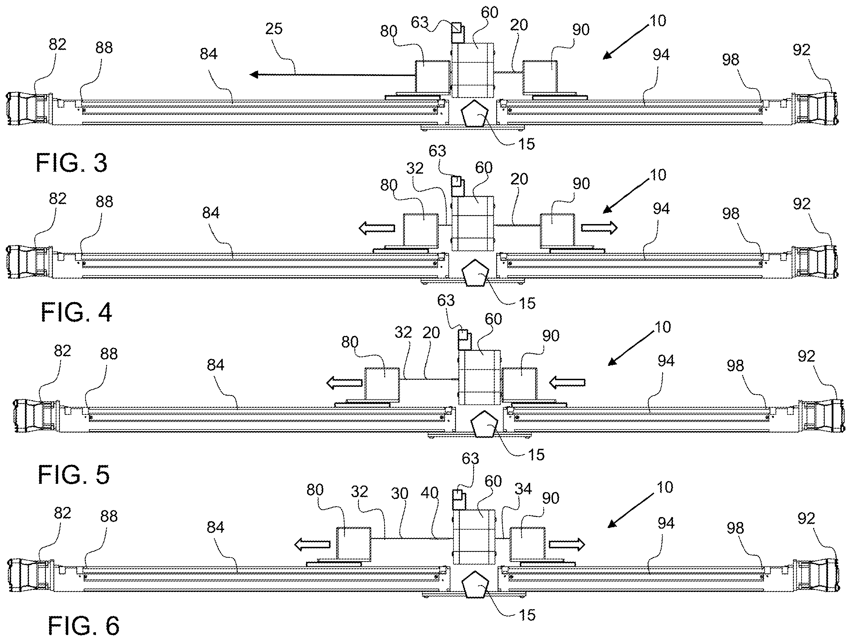

Figure 3 shows the preform tube retained between a first tube retainer and a second tube retainer and a radial compression heater configured around the preform tube.
Figure 4 shows the preform tube being stretched by the two retainers moving apart from each other, wherein the heated portion of the preform tube is stretched to form a first stretched portion.
Figure 5 shows the preform tube being moved with respect to the radial compression heater to locate a new or second portion of the preform tube within the radial compression heater.
Figure 6 shows this second portion of the preform tube being stretched by the two retainers moving apart from each other to form a parison tube having a balloon portion configured between the first stretched portion and the second stretched portion.

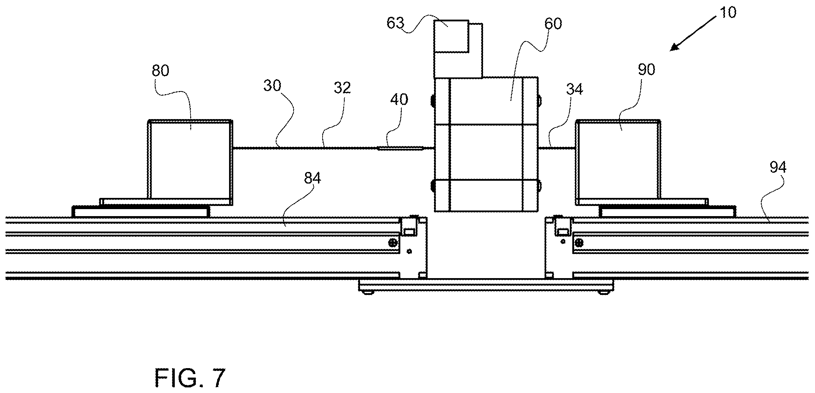

Figure 7 shows an enlarged view of FIG. 6, wherein the balloon portion 40 is configured between the first stretched portion and the second stretched portion of the parison tube.

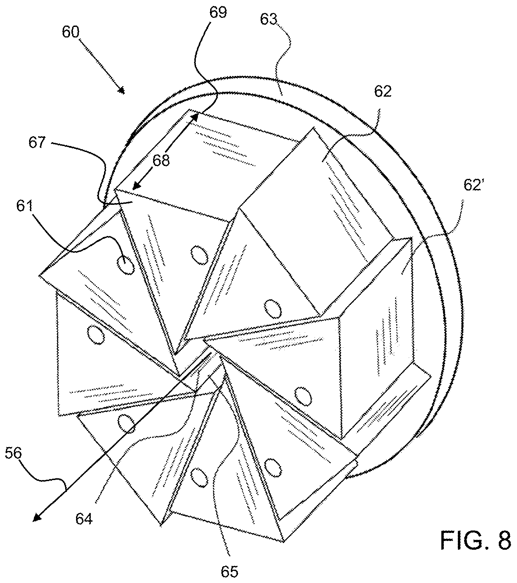

Figure 8 shows an exemplary radial compression heater having a plurality of compression dies that move with respect to each other to form a central cavity that closes down onto a preform tube to heat said preform tube for stretching.

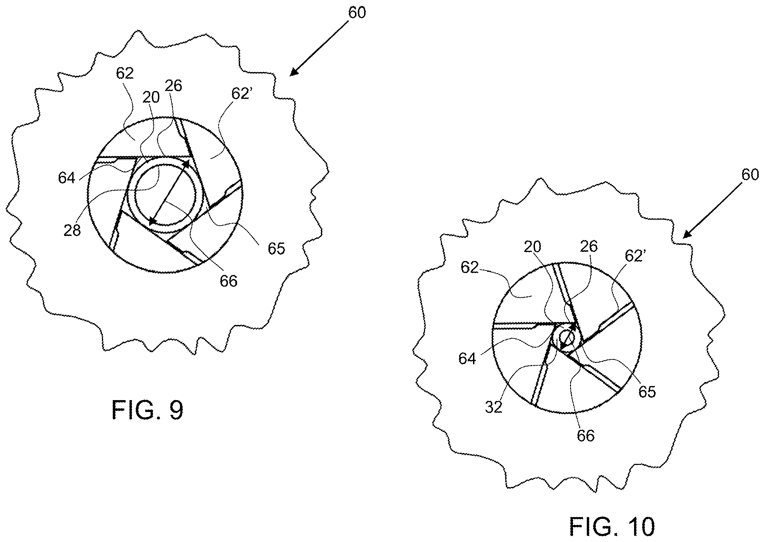

Figure 9 shows a first end view of a radial compression heater configured around a preform tube to heat said preform tube.
Figure 10 shows a first end view of the radial compression heater shown in FIG. 9 after the preform tube has been stretched, wherein the compression dies move radially inward with the necking tube to continue to heat the tube by conduction during the stretching process.

Corresponding reference characters indicate corresponding parts throughout the several views of the figures. The figures represent an illustration of some of the embodiments of the present invention and are not to be construed as limiting the scope of the invention in any manner. Some of the figures may not show all of the features and components of the invention for ease of illustration, but it is to understood that where possible, features and components from one figure may be an included in the other figures. Further, the figures are not necessarily to scale, some features may be exaggerated to show details of particular components. Therefore, specific structural and functional details disclosed herein are not to be interpreted as limiting, but merely as a representative basis for teaching one skilled in the art to variously employ the present invention.

### DETAILED DESCRIPTION OF THE ILLUSTRATED EMBODIMENTS

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Also, use of "a" or "an" are employed to describe elements and components described herein. This is done merely for convenience and to give a general sense of the scope of the invention. This description should be read to include one or at least one and the singular also includes the plural unless it is obvious that it is meant otherwise.

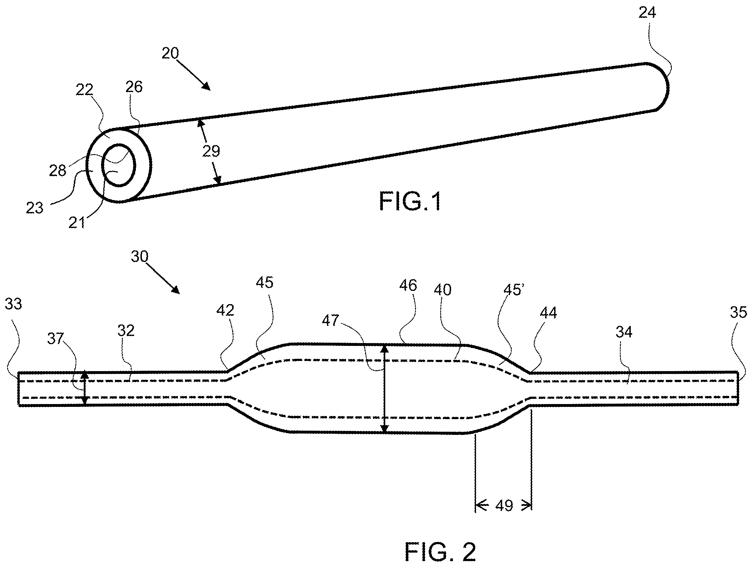

Referring now to FIGS. 1 and 2, an exemplary parison tube 30, as shown in FIG. 2, is formed from a preform tube 20, shown in FIG. 1. The preform tube 20 is stretched or necked down to form a first stretched portion 32 and a second stretched portion 34 with a balloon portion 40 configured therebetween. FIG. 1 shows a perspective view of an exemplary preform tube that is used to form a parison tube having a balloon portion. The preform tube has a wall 23 thickness from an outer surface 26 to an inner surface 28, a preform tube diameter 29 and a length from a first end 22 to a second end 24. The exemplary preform tube is cylindrical in shape, a cylinder that forms a conduit 21. The parison tube 30 has a balloon portion 40 that has a larger diameter 47 than the diameter 37 of the first and second stretched portions. The balloon portion 40 has tapered ends 45, 45' that taper down to the first stretched portion 32 and second stretched portion 34, respectively. The balloon portion 40 and has a length from first end 42 to the second end 44 of the balloon portion. The length 49 of the tapered ends may be minimized by uniform heating of the preform with the radial compression heater. The balloon portion has an outer surface 46. The parison tube may be cut to a desired length from a first stretched extended end 33 to a second stretched extended end 35.

Referring now to FIGS. 3 to 6, an exemplary parison former 10 forms a parison tube 30 by selectively stretching the preform tube 20. As shown in FIG. 3, the preform tube 20 is retained between a first tube retainer 80 and a second tube retainer 90. The radial compression heater 60 is configured around the preform tube 20 to heat a first portion of the preform tube to enable stretching. The radial compression heater has an actuator 63 that closes the compression dies onto the outer surface of the preform tube to heat the preform tube through conduction, direct contact with the preform tube. After the tube is effectively heated, the preform tube is stretched, as shown in FIG. 4, to form a first stretched portion 32. The first tube retainer 80 and a second tube retainer 90 are moved apart from one another on a first rail 84 and a second rail 94, respectively. Note that moving apart may involve both tube retainers moving, or only one moving while the other remains stationary. A first tube retainer may be moved by a first tube retainer actuator 82, such as a servo motor that moves the first tube retainer along the first rail 84. Likewise, the second tube retainer 90 be moved by a second tube retainer actuator 92, such as a servo motor that moves the second tube retainer along the second rail 94. A servo motor may provide for very smooth motion and may enable the rate of stretching to be customized to produce uniform stretched portion of the preform tube.

As shown in FIG. 5, the preform tube 20 is moved with respect to the radial compression heater to locate a new or second portion of the preform tube within the radial compression heater 60. A controller 15 may control the motion of the components to locate the second portion of the tube in the radial compression heater. The radial compression heater may be configured to move along the length axis 25, or length of the preform tube, or the first tube retainer and the second tube retainer may be configured to move the preform tube such that a second portion of the preform tube is configured within the radial compression heater. The radial compression heater may be on a rail, which may be the same rail as the first rail or second rail. A single rail may be used for example extending from a first rail end 88 to a second rail end 98. The die actuator will close down around this second portion of tube to apply heat again through conduction. The first tube retainer 80 and the second tube retainer 90 are moved apart from one another to produce a second stretched portion 34 and a balloon portion 40 between the first stretched portion 32 and said second stretched portion 34 of the parison tube 30, as shown in FIG. 6. Only one of the tube retainers, the second tube retainer 90 may be moved to move the first and second tube retainers apart from each other to form the second stretched portion 34.

As shown in FIG. 7, the balloon portion 40 is configured between the first stretched portion 32 and the second stretched portion 34 of the parison tube 30. The parison tube is formed by iterative stretching and moving the perform tube by the first tube retainer 80 and the second tube retainer 90 to configured a second portion of the tube in the radial compression heater 60 after a first portion has been stretched to form the first stretched portion 32.

As shown in FIG. 8, an exemplary radial compression heater 60 has a plurality of compression dies 62, 62' arranged about a central axis 56, that move with respect to each other to form a central cavity 65, of the working surfaces 64, that close down onto a preform tube to heat said preform tube for stretching. The compression dies, or central cavity 65 formed therefrom, have a first end 67 and a second end 69 and a length 68 to receive a preform tube. One or more heaters 61 may be configured to heat the compression dies.

As shown in FIG. 9, an exemplary radial compression heater 60 is configured around a preform tube 20 to heat said preform tube. The working surfaces 64 of the compression dies 62 form a central cavity 65, having a central cavity diameter 66, that is in contact with the outer surface 26 of the preform tube. The preform tube is heated by conduction and direct contact with the working surfaces 64.

As shown in FIG. 10, the exemplary radial compression heater 60 is configured around a first stretched portion 32 of a preform tube 20 to continue to heat said preform tube as it is being stretched. The working surfaces 64 of the compression dies 62 form a central cavity 65, having a central cavity diameter 66, that is in contact with the outer surface 26 of the preform tube. The compression dies may be forced inward by a die actuator to reduce the central cavity diameter and press the working surfaces into contact with the outer surface of the preform tube as it is stretched and reduced in diameter, as shown.

## Claims

1. A parison tube former (10) comprising:
a) a first tube retainer (80);
b) a second tube retainer (90);
c) a radial compression heater (60) comprising:
i) a plurality of compression dies (62, 62') each having a working surface (64),
wherein the plurality of compression dies (62, 62') are arranged radially with respect to each other to form a central cavity (65) defined by said working surfaces (64), wherein the
central cavity (65) has a central cavity diameter;
ii) a heater (61) configured in one or more compression dies and configured to heat the compression dies (62, 62');
iii) a die actuator configured to move the plurality of compression dies (62, 62') inward to reduce the central cavity diameter;
d) a preform tube (20) extending through the central cavity (65) of the radial compression heater (60) and having a length between the first tube retainer (80) and the second tube retainer (90);
wherein the radial compression heater (60) is configured to heat a portion of the length of the preform tube (20) between the first tube retainer (80) and the second tube retainer (90),
wherein the plurality of compression dies (62, 62') are configured to close down onto the outer surface of the preform tube (20), wherein the working surface (64) of said plurality of compression dies (62, 62') are configured to contact the preform tube to heat a portion of said length of the preform tube via conduction (20);
and wherein the first tube retainer (80) and second tube retainer (90) are configured to move apart from each other to stretch the preform tube (20) and form a parison tube (30) having a balloon portion (40) configured between a first stretched portion (32) and a second stretched portion (34).

2. The parison tube former (10) of claim 1, wherein the first tube retainer (80) and the second tube retainer (90) move in opposing directions to stretch the preform tube (20) to form the first stretched portion (32).

3. The parison tube former (10) of claim 1, wherein only one of the first tube retainer (80) and the second tube retainer (90) moves to stretch the preform tube (20) to form the second stretched portion (34).

4. The parison tube former (10) of claim 1, wherein the working surfaces (64) of the plurality of dies (62, 62') are forced by the die actuator radially inward to reduce the central cavity diameter while the preform tube (20) is stretched.

5. The parison tube former (10) of claim 1, configured for a preform tube (20) comprising a thermoplastic polymer.

## Patentansprüche

1. Vorformlingsrohrformer (10), umfassend:
a) einen ersten Rohrhalter (80);
b) einen zweiten Rohrhalter (90);
c) eine radiale Druckheizung (60), umfassend:
i) eine Vielzahl von Druckstempeln (62, 62'), die jeweils eine Arbeitsoberfläche (64) aufweisen,
wobei die Vielzahl von Druckstempeln (62, 62') radial in Bezug zueinander angeordnet sind, um einen zentralen Hohlraum (65) zu bilden, der durch die Arbeitsoberflächen (64) definiert ist, wobei der zentrale Hohlraum (65) einen zentralen Hohlraumdurchmesser aufweist;
ii) eine Heizung (61), die in einem oder mehreren Druckstempeln ausgebildet ist und ausgebildet ist, um die Druckstempel (62, 62') zu erhitzen;
iii) eine Stempelbetätigungsvorrichtung, die ausgebildet ist, um die Vielzahl von Druckstempeln (62, 62') nach innen zu bewegen, um den zentralen Hohlraumdurchmesser zu verringern;
d) ein Vorformrohr (20), das sich durch den zentralen Hohlraum (65) der radialen Druckheizung (60) erstreckt und eine Länge zwischen dem ersten Rohrhalter (80) und dem zweiten Rohrhalter (90) aufweist;
wobei die radiale Druckheizung (60) ausgebildet ist, um einen Abschnitt der Länge des Vorformrohres (20) zwischen dem ersten Rohrhalter (80) und dem zweiten Rohrhalter (90) zu erhitzen,
wobei die Vielzahl von Druckstempeln (62, 62') ausgebildet ist, um auf die Außenoberfläche des Vorformrohres (20) zu schließen, wobei die Arbeitsoberfläche (64) der Vielzahl von Druckstempeln (62, 62') ausgebildet ist, um das Vorformrohr zu berühren, um einen Abschnitt der Länge des Vorformrohres durch Wärmeleitung (20) zu erhitzen; und
wobei der erste Rohrhalter (80) und der zweite Rohrhalter (90) ausgebildet sind, um sich voneinander weg zu bewegen, um das Vorformrohr (20) zu dehnen und ein Vorformlingsrohr (30) zu bilden, das einen Ballonabschnitt (40) aufweist, der zwischen einem ersten gedehnten Abschnitt (32) und einem zweiten gedehnten Abschnitt (34) ausgebildet ist.

2. Vorformlingsrohrformer (10) nach Anspruch 1, wobei sich der erste Rohrhalter (80) und der zweite Rohrhalter (90) in entgegengesetzte Richtungen bewegen, um das Vorformrohr (20) zu dehnen, um den ersten gedehnten Abschnitt (32) zu bilden.

3. Vorformlingsrohrformer (10) nach Anspruch 1, wobei sich nur einer von dem ersten Rohrhalter (80) und dem zweiten Rohrhalter (90) bewegt, um das Vorformrohr (20) zu dehnen um den zweiten gedehnten Abschnitt (34) zu bilden.

4. Vorformlingsrohrformer (10) nach Anspruch 1, wobei die Arbeitsoberflächen (64) der Vielzahl von Stempeln (62, 62') durch die Stempelbetätigungsvorrichtung radial nach innen gedrängt werden, um den zentralen Hohlraumdurchmesser zu verringern, während das Vorformrohr (20) gedehnt wird.

5. Vorformlingsrohrformer (10) nach Anspruch 1, der für ein Vorformrohr (20) ausgebildet ist, das ein thermoplastisches Polymer umfasst.

## Revendications

1. Dispositif de formation de tube de paraison (10) comprenant :
a) un premier dispositif de retenue de tube (80) ;
b) un deuxième dispositif de retenue de tube (90) ;
c) un dispositif de chauffage à compression radiale (60) comprenant :
i) une pluralité de matrices de compression (62, 62') présentant chacune une surface de travail (64),
dans lequel la pluralité de matrices de compression (62, 62') sont agencées radialement les unes par rapport aux autres pour former une cavité centrale (65) définie par lesdites surfaces de travail (64), dans lequel la cavité centrale (65) présente un diamètre de cavité centrale ;
ii) un dispositif de chauffage (61) configuré dans une ou plusieurs matrices de compression et configuré pour chauffer les matrices de compression (62, 62') ;
iii) un actionneur de matrices configuré pour déplacer la pluralité de matrices de compression (62, 62') vers l'intérieur pour réduire le diamètre de cavité centrale ;
d) un tube de préforme (20) s'étendant à travers la cavité centrale (65) du dispositif de chauffage à compression radiale (60) et présentant une longueur entre le premier dispositif de retenue de tube (80) et le deuxième dispositif de retenue de tube (90) ;
dans lequel le dispositif de chauffage à compression radiale (60) est configuré pour chauffer une partie de la longueur du tube de préforme (20) entre le premier dispositif de retenue de tube (80) et le deuxième dispositif de retenue de tube (90),
dans lequel la pluralité de matrices de compression (62, 62') sont configurées pour se refermer sur la surface externe du tube de préforme (20), dans lequel la surface de travail (64) de ladite pluralité de matrices de compression (62, 62') est configurée pour entrer en contact avec le tube de préforme pour chauffer une partie de ladite longueur du tube de préforme par conduction (20) ; et dans lequel le premier dispositif de retenue de tube (80) et le deuxième dispositif de retenue de tube (90) sont configurés pour s'écarter l'un de l'autre pour étirer le tube de préforme (20) et former un tube de paraison (30) présentant une partie de ballonnet (40) configurée entre une première partie étirée (32) et une deuxième partie étirée (34).

2. Dispositif de formation de tube de paraison (10) selon la revendication 1, dans lequel le premier dispositif de retenue de tube (80) et le deuxième dispositif de retenue de tube (90) se déplacent dans des directions opposées pour étirer le tube de préforme (20) pour former la première partie étirée (32).

3. Dispositif de formation de tube de paraison (10) selon la revendication 1, dans lequel seul l'un du premier dispositif de retenue de tube (80) et du deuxième dispositif de retenue de tube (90) se déplace pour étirer le tube de préforme (20) pour former la deuxième partie étirée (34).

4. Dispositif de formation de tube de paraison (10) selon la revendication 1, dans lequel les surfaces de travail (64) de la pluralité de matrices (62, 62') sont forcées par l'actionneur de matrice radialement vers l'intérieur pour réduire le diamètre de cavité centrale pendant que le tube de préforme (20) est étiré.

5. Dispositif de formation de tube de paraison (10) selon la revendication 1, configuré pour un tube de préforme (20) comprenant un polymère thermoplastique.
